(19) Europäisches Patentamt
European Patent Office
Office européen des brevets

(11) Numéro de publication: **0 003 200**
**B1**

(12) # FASCICULE DE BREVET EUROPÉEN

(45) Date de publication du fascicule du brevet: **13.08.86**

(51) Int. Cl.⁴: **A 61 K 31/445,** C 07 D 401/04

(21) Numéro de dépôt: **79400020.8**

(22) Date de dépôt: **09.01.79**

(54) **Dérivés du tétrahydro pyridinyl-indole et leurs sels, pour leur utilisation dans une méthode de traitement thérapeutique du corps humain ou animal, et compositions pharmaceutiques les renfermant.**

(30) Priorité: **16.01.78 FR 7801084**

(43) Date de publication de la demande:
**25.07.79 Bulletin 79/15**

(45) Mention de la délivrance du brevet:
**13.08.86 Bulletin 86/33**

(84) Etats contractants désignés:
**BE CH DE GB IT NL**

(56) Documents cité:
**FR-A-2 293 931**
**FR-A-2 328 468**

**JOURNAL OF ORGANIC CHEMISTRY, vol. 40, nr. 17, 1975 "Pharma research Canada LTD"**

(73) Titulaire: **ROUSSEL- UCLAF, 102, route de Noisy Boîte postale no.9, F-93230 Romainville (FR)**

(72) Inventeur: **Nedelec, Lucien, 45, boulevard de l'Ouest, F-93340 Le Raincy (FR)**
Inventeur: **Guillaume, Jacques, 11, Allée Hélène Boucher, F-93270 Sevran (FR)**
Inventeur: **Dumont, Claude, 33, rue du Maréchal Vaillant, F-94130 Nogent- sur- Marne (FR)**

(74) Mandataire: **Vieillefosse, Jean- Claude, Département des Brevets ROUSSEL UCLAF B.P. no 9, F-93230 Romainville (FR)**

**EP 0 003 200 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne des dérivés du tétrahydropyridinyl indole et leurs sels, pour leur utilisation dans une méthode de traitement thérapeutique du corps humain ou animal, et les compositions pharmaceutiques les renfermant.

L'article de "Pharma Research Canada Ltd." paru dans le Journal of organic chemistry, vol. 40, no 17, p. 2525—2529 avait déjà décrit d'un point de vue chimique un certain nombre de dérivés du tétrahydropyridinyl-indole. Les brevets français no 2.293.931 et le certificat d'addition no 2.328.468 avaient déjà décrit des dérivés du pipéridyl indole doués de propriétés antidépressives et antiémétiques.

L'invention a pour objet les composés chimiques constitués par les dérivés du tétrahydropyridinyl indole, répondant à la formule générale:

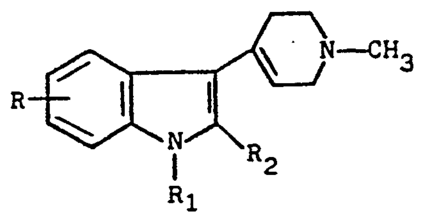

(I)

dans laquelle R représente un atome d'hydrogène ou un radical méthoxy, $R_1$ et $R_2$, identiques ou différents, représentent un atome d'hydrogène ou un radical méthyle, ainsi que par les sels d'addition avec les acides pharmaceutiquement acceptables, desdits dérivés, pour leur utilisation dans une méthode de traitement thérapeutique du corps humain ou animal.

Les sels d'addition avec les acides pharmaceutiquement acceptables peuvent être, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique alcanosulfoniques, tels que l'acide méthane sulfonique et arylsulfoniques, tels que l'acide benzène sulfonique.

Parmi les composés chimiques selon l'invention, on peut citer notamment, les dérivés du tétrahydro pyridinyl indole répondant à la formule I ci-dessus dans laquelle R a la signification déjà indiquée et $R_1$ et $R_2$ représentent un atome d'hydrogène, ainsi que leurs sels d'addition avec les acides pharmaceutiquement acceptables.

Parmi les composés chimiques selon l'invention, on retient plus particulièrement:

—le 5-méthoxy 3-(1-méthyl 1,2,3,6-tétrahydro pyridin- 4-yl) 1H-indole, ainsi que ses sels d'addition avec les acides pharmaceutiquement acceptables;

—le 3-(1-méthyl 1,2,3,6-tétrahydro pyridin-4-yl) 1H-indole, ainsi que ses sels d'addition avec les acides pharmaceutiquement acceptables.

Les dérivés du tétrahydro pyridinyl indole de formule (I) et leurs sels possèdent de très intéressantes propriétés pharmacologiques, illustrées plus loin dans la partie expérimentale; ils sont doués, notamment, de remarquables propriétés à la fois antidépressives et neuroleptiques, ainsi que de propriétés antiémétiques.

En raison de ces propriétés, les composés chimqes, selon la présente invention, trouvent par exemple leur emploi dans le traitement des troubles psychiques, des troubles du comportement, des troubles caractériels ainsi que dans le traitement des vomissements en nausées de toutes origines.

La dose usuelle, variable selon le produit utilisé, le sujet traité et l'affection en cause, peut être par exemple de 5 mg à 200 mg par jour, par voie orale, chez l'homme.

L'invention a enfin pour objet les compositions pharmaceutiques qui renferment au moins un dérivé précité, ou l'un de ses sels d'addition avec les acides pharmaceutiquement acceptables, à titre de principe actif, ainsi qu'un excipient pharmaceutique inerte, à l'exception de l'éthanol.

Pour leur utilisation dans une méthode de traitement thérapeutique du corps humain ou animal, les dérivés répondant à la formule I et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les dérivés du tétrahydro-pyridinyl-indole répondant à la formule générale I peuvent être préparés comme indiqué dans J.Org.Chem. Vol. 40, No. 17, 1975, pages 2525 à 2529.

Les sels des dérivés du tétrahydropyridinyl-indole, répondant à la formule générale I, lorsqu'ils ne sont pas connus, peuvent être préparés en faisant réagir en proportions sensiblement stoéchiométriques, un acide minéral ou organique avec lesdits dérivés.

Les préparations du succinate acide du 5-méthoxy 3-(1-méthyl 1,2,3,6-tétrahydro pyridin-4-yl) 1H-indole et du chlorhydrate du 3-(1-méthyl 1,2,3,6-tétrahydro pyridin-4-yl) 1H-indole, sont données à titre d'exemple dans la partie expérimentale.

Les exemples suivants illustrent l'invention sans toutefois la limiter.

Exemple 1

succinate acide du 5-méthoxy 3-(1-méthyl 1,2,3,6 tétrahydropyridin-4-yl) 1H-indole.

Stade A: préparation du 5-méthoxy 3-(1-méthyl 1,2,3,6 tétrahydropyridin-4-yl) 1H-indole.

En opérant selon le procédé indiqué dans J.Org.Chem.Vol. 40 no 17 1975, p2525, par condensation de la 1-méthyl pipéridone sur le 5-méthoxy indole, dans l'acide acétique au reflux en présence d'acide phosphorique, on obtient le produit recherché, fondant à 235°C.

Stade B: succinate acid du 5-méthoxy 3-(1-méthyl 1,2,3,6 tétrahydropyridin-4-yl) 1H-indole.

On dissout 3,5 g du produit préparé au stade A dans une solution refermant 1,7 g d'acide succinique dans 25 cm3 de méthanol. On maintient sous agitation pendant 30 minutes à la température ambiante, puis 2 heures dans un bain de glace. On filtre, rince au méthanol et sèche sous pression réduite à 50°C.. On obtient 4,17 g du produit recherché, fondant à 153°C.

ANALYSE: $C_{19}H_{24}N_2O_5$ = 360,418
Calculé: C% 63,32 H% 6,71 N% 7,77
Trouvé: 63,3 6,9 7,7

Exemple 2

chlorhydrate de 3-(1-méthyl 1,2,3,6 tétrahydropyridin-4-yl) 1H-indole.

Stade A: 3-(1-méthyl 1,2,3,6 tétrahydropyridin-4-yl) 1H-indole.

En opérant selon le procédé indiqué dans J. Org. Chem. Vol. 40 no. 17, 1975, p 2525, par condensation de la 1-méthyl pipéridone sur l'indole dans l'acide acétique au reflux, en présence d'acide phosphorique, on obtient le produit recherché, fondant à 228~229°C.

Stade B: chlorhydrate de 3-(1-méthyl 1,2,3,6-tétrahydropyridin-4-yl) 1H-indole.

On met en suspension 4,12 g du produit préparé au stade A, dans 80 cm3 d'éthanol. On glace et ajoute, sous agitation, une solution d'acide chlorhydrique dans l'éthanol jusqu'à l'obtention de pH 1. On maintient sous agitation pendant une heure à 0°C., filtre et rince à l'éthanol. On obtient 4,6 g de produit brut que l'on dissout dans 100 cm3 de méthanol au reflux. On filtre à chaud et amorce la cristallisation. On glace pendant 2 hours, filtre et rince au méthanol. On obtient ainsi 3,04 g de produit recherché, fondant à 241~243°C.

ANALYSE; $C_{14}H_{16}N_2$, HCl = 248,762
Calculé: C% 67,59 H% 6,88 Cl% 14,25 N% 11,26
Trouvé: 67,7 7,0 14,2 10,9

Exemple 3

On a préparé des comprimés répondant à la formule:
—Composé de l'exemple 1 .... 25 mg
—Excipient q.s. pour un comprimé terminé à .... 200 mg

Exemple 4

On a préparé un soluté injectable répondant à la formule:
—Composé de l'exemple 2 .... 25 mg
—Excipient aqueux stérile q.s.p. .... 2 ml

ETUDE PHARMACOLOGIQUE.

1) Potentialisation des stéróotypies à l'amphétamine:

Les essais sont réalisés sur des lots de 5 rats mâles, de 150 à 180 g. Chaque animal est placé individuellement dans une cage grillagés (29 × 25 × 17 cm) contenant quelques débris de frisure de bois.

Un délai d'une heure est observé entre l'administration du composé étudié et l'injection, par voie intrapéritonéale, de 5 mg/kg de sulfate de dexamphé tamine. Le comportement des animaux est noté ensuite toutes les 1/2 heures pendant 5 heures avec la cotation préconisée par HALLIWELL et Coll. (Brit. J. Pharmacol. 1964, *23*, 330—350). L'animal est endormi (0), il est éveillé mais immobile (1), il tourne dans la cage (2), il en renifle le couvercle (3), il en lèche les parois (4), il touche les copeaux ou les barreaux de la cage avec les dents (5), il mord les copeaux ou les barreaux de la cage (6).

L'intensité des stéréotypies est exprimée sous la forme d'un score compris entre 0 et 30 correspondant à la somme des valeurs obtenues sur les 5 rats d'un lot à chaque temps. La somme des scores relevés en 5 heures est calculée.

Les composés sont administrés par voie intrapéritonéale.

La dose des composés qui augmente d'environ 100% la somme des scores en 5 houres est de 2 mg/kg pour le composé de l'exemple 1 et de 20 mg/kg pour le composé de l'exemple 2.

2) Antagonisme à l'égard des stéréotypies à l'apomorphine.

Les essais sont réalisés sur des lots de 5 rats selon un protocole inspiré de JANSSEN et coll. (Arzneim. Forsch. 1965, 15, 104—117; 1967, 17, 841—854). Chaque animal est placé individuellement dans une boite en plexiglas (20 × 10 × 10 cm; NICOLET) dont le fond est recouvert d'une mince couche de frisure de bois.

Une dose de 1,5 mg/kg de chlorhydrate d'apomorphine est injectée par voie intraveineuse, 1/2h. après l'administration intrapéritonéale du composé étudié.

Les animaux sont observés pendant 1 mn, 15 mn après l'injection de l'apomorphine. Les mouvements stéréotypés de la sphère buccale sont évalués selon BOISSIER et SIMON (Thérapie, 1970, 25, 939—949): pas de réaction caractéristique (0), quelques reniflements, lèchages et mâchonnements (1), reniflements intenses et léchages continus (2), mâchonnements continus (3).

L'intensité de stéréotypies est exprimés sous la forme d'un score compris entre 9 et 15, correspondant à la somme des valeurs obtenues sur les 5 rats d'un lot, 15 minutes après l'injection de l'apomorphine.

La dose de composé de l'exemple 2 que réduit d'environ 50% la somme des scores est de 20 mg/kg.

3) Potentialisation de la toxicité de l'yohimbine

L'esai est réalisé selon la technique de QUINTON (Brit. J. Pharmacol. 1963, *21*, 51).

Une dose sub-léthale de 30 mg/kg de chlorhydrate d'yohimbine est injectée par voie intrapéritonéale à des lots de 10 souris mâles de 22 à 24 g.

L'injection du composé étudié a lieu par voie intrapéritonéale 1 h avant l'injection de l'yohimbine. La mortalité est relevée 24 h après l'injection de cette dernière.

Le composé de l'exemple 1 potentialise la toxicité de l'yohimbine à la dose de 10 mg/kg et le composé de l'exemple 2, à la dose de 35 mg/kg.

4) Antagonisme de la catalepsie induite par la prochlorpemazine.

Les essais sont réalisé sur des lots de 5 rats mâles de 100 g environ.

Le composé étudié est administré par vote intrapéritonéale simultanément avec une dose de 15 mg/kg de prochlorpemazine par voie intrapéritonéale.

La catalepsie est appréciée toutes les heures pendant 7 heurs suivant le test de croisement des pattes homolatérales (BOISSIER, SIMON, Therapie, 1963, *18*, 1257—1277) avec la cotation suivante: l'animal refuse le croisement des pattes antérieures avec les pattes postérieures homolatérales (0), il accepte le croisement recherché seulement d'un côté (0,5), il accepte le croisement des deux côtés (1).

Le composé de l'exemple 2 s'oppose à la catalepsie induite par la prochlorpemazine, à la dose de 20 mg/kg.

5) Activité antiémétique:

L'antagonisme vis-à-vis des vomissements provoqués par l'apomorphine est étudié chez le chien (CHEN et ENSOR J. Pharmac. exp. Thérap. 1959, *93*, 245—250).

Le nombre de vomissements provoqués par une injection sous-cutanée de 0,1 mg/kg de chlorhydrate d'apomorphine est déterminé sur chaque animal 8 jours avant l'essai.

Le composé étudié, mis en solution aqueuse, est administré par voie sous-cutanée à des doses variables une demi -heure avant le chlorhydrate d'apomorphine.

Le composé de l'exemple 1 réduit d'environ 50% les vomissements provoqués par l'apomorphine à la dose de 0,08 mg/kg, le composé de l'exemple 2 à la dose de 0,05 mg/kg.

6) Etude de la toxicité aigüe:

La toxicité aigüe est déterminée sur des lots de dix souris pesant 20 g environ, auxquelles on administre, par voie intrapéritonéale, des doses croissantes du composé étudié.

La mortalité est relevée 48 heures après l'administration du composé.

Les doses létales 50 ($DL_{50}$) approchées des composés des exemples 1 et 2 sont respectivement égales à 150 et à 120 mg/kg.

**Revendications**

1. Composés chimiques constitués par les dérivés du tétrahydropyridinyl-indole répondant à la formule générale:

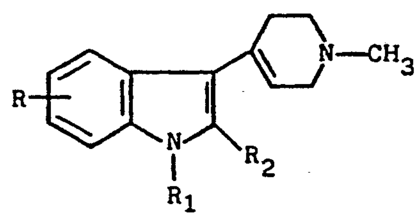

(I)

dans laquelle R représente un atome d'hydrogène ou un radical méthoxy, $R_1$ et $R_2$, identiques ou différents,

représentent un atome d'hydrogène ou un radical méthyle, ainsi que par les sels d'addition avec les acides pharmaceutiquément acceptables, desdits dérivés, pour leur utilisation dans une méthode de traitement thérapeutique du corps humain ou animal.

2. Composés chimiques selon la revendication 1, pour leur utilisation comme antidépresseurs chez l'homme ou l'animal.

3. Composés chimiques selon la revendication 1, pour leur utilisation comme neuroleptiques chez l'homme ou l'animal.

4. Composés chimiques selon la revendication 1, pour leur utilisation comme antiémétiques chez l'homme ou l'animal.

5. Composés chimiques selon la revendication 1, destinés au traitement des troubles psychiques, des troubles du comportement et des troubles caractériels chez l'homme ou l'animal.

6. Composés chimiques selon la revendication 1, destinés au traitement des vomissements et nausées de toutes origines chez l'homme ou l'animal.

7. Composés chimiques, selon l'une des revendications 1 à 6, caractérisés en ce qu'ils sont constitués par les dérivés du tétrahydropyridinyl-indole répondant à la formule I de la revendication 1, dans laquelle R a la signification indiquée et $R_1$ et $R_2$ représentent un atome d'hydrogène, ainsi que par les sels d'addition avec les acides pharmaceutiquement acceptables desdits dérivés.

8. Composés chimiques, selon l'une des revendications 1 à 6, caractérisés en ce qu'il est constitué par le 5-méthoxy 3-(1-méthyl 1,2,3,6-tétrahydropyridin-4-yl)1H-indole, ainsi que par ses sels d'addition avec les acides pharmaceutiquement acceptables.

9. Composé chimique, selon l'une des revendications 1 à 6, caractérisé en ce qu'il est constitué par le 3-(1-méthyl 1,2,3,6-tétrahydropyridin-4-yl)1H-indole, ainsi que par ses sels d'addition avec les acides pharmaceutiquement acceptables.

10. Compositions pharmaceutiques, caractérisées en ce qu'elles renferment, à titre de principe actif, l'un au moins des composés chimiques définis dans l'une des revendications 1 à 9, ainsi qu'un excipient pharmaceutique inerte, à l'exception de l'éthanol.

**Patentansprüche**

1. Chemische Verbindungen, bestehend aus den Tetrahydropyridinyl-indol-derivaten der allgemeinen Formel

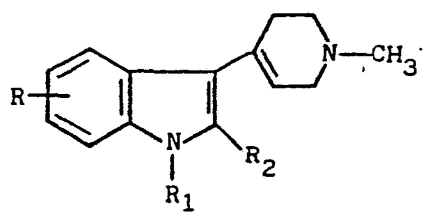

(I)

worin R ein Wasserstoffatom oder eine Methoxygruppe bedeutet, $R_1$ und $R_2$, die gleich oder verschieden sein können, ein Wasserstoffatom oder einen Methylrest darstellen sowie aus den Additionssalzen dieser Derivate mit pharmazeutisch verträglichen Säuren zur Anwendung in einem Verfahren zur therapeutschen Behandlung des menschlischen oder tierischen Körpers.

2. Chemische Verbindungen gemäß Anspruch 1 zur Verwendung als Antidepressiva bei Mensch oder Tier.

3. Chemische Verbindungen gemäß Anspruch 1 zur Verwendung als Neuroleptika bei Mensch oder Tier.

4. Chemische Verbindungen gemäß Anspruch 1 zur Verwendung als Antemica-bei Mensch oder Tier.

5. Chemische Verbindungen gemäß Anspruch 1 zur Behandlung von psychischen Störungen, Verhaltensstörungen und Charakterstörungen bei Mensch oder Tier.

6. Chemische Verbindungen gemäß Anspruch 1 zur Behandlung von Erbrechen und Übelkeit jeglichen Ursprungs bei Mensch oder Tier.

7. Chemische Verbindungen gemäß einem der Ansprüche 1 bis 6, dadurch gekennzeichnet, daß sie aus den Tetrahydropyridinylindol-derivaten der Formel (I) gemäß Anspruch 1, worin R die angegebene Bedeutung besitzt und $R_1$ und $R_2$ ein Wasserstoffatom bedeuten sowie aus den Additionssalzen dieser Derivate mit pharmazeutisch verträglichen Säuren bestehen.

8. Chemische Verbindung gemäß einem der Ansprüche 1 bis 6, dadurch gekennzeichnet, daß sie aus dem 5-Methoxy-3-(1-methyl-1,2,3,6-tetrahydropyridin-4-yl)-1H-indol sowie aus seinen Additionssalzen mit pharmazeutisch verträglichen Säuren besteht.

9. Chemische Verbindung gemäß der Ansprüche 1 bis 6, dadurch gekennzeichnet, daß sie aus dem 3-(1-Methyl-1,2,3,6-tetrahydropyridin-4-yl)-1H-indol sowie aus seinen Additionssalzen mit pharmazeutisch verträglichen Säuren besteht.

10. Pharmazeutische Zusammensetzungen, dadurch gekennzeichnet, daß sie als Wirkstoff zumindest eine der gemäß einem der Ansprüche 1 bis 9 definierten chemischen Verbindungen sowie einen inerten pharmazeutischen Exzipienten mit Ausnahme von Äthanol enthalten.

**Claims**

1. Chemical compounds constituted by the derivatives of tetrahydropyridinylindole, responding to the general formula:

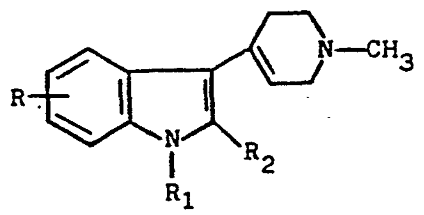

(I)

in which R represents a hydrogen atom or a methoxy radical, $R_1$ and $R_2$, which may be identical or different, represent each a hydrogen atom or a methyl radical, as well as by the salts of addition with pharmaceutically acceptable acids of the said derivatives, for their use in a therapeutic treatment method of the human or animal body.

2. Chemical compounds according to Claim 1, for their use as anti-depressants in man or animal.

3. Chemical compounds according to Claim 1, for their use as neuroleptics in man or animal.

4. Chemical compounds according to Claim 1, for their use as anti-emetics in man or animal.

5. Chemical compounds according to Claim 1, intended for the treatment of psychical disorders, behavioural disorders and emotional disturbances in man or animal.

6. Chemical compounds according to Claim 1, intended for the treatment of vomitting and nausea from all causes in man or animal.

7. Chemical compounds according to one of the Claims 1 to 6, characterized in that they are constituted by the derivatives of tetrahydropyridinylindole responding to formula I of Claim 1, in which R has the significance indicated and $R_1$ and $R_2$ each represent a hydrogen atom, as well as by the salts of addition with pharmaceutically acceptable acids of the said derivatives.

8. Chemical compound, according to one of the Claims 1 to 6, characterized in that it is constituted by 5-methoxy-3-(1-methyl-1,2,3,6-tetrahydropyridin-4-yl)-1H-indole, as well as by its salts of addition with pharmaceutically acceptable acids.

9. Chemical compound, according to one of the Claims 1 to 6, characterized in that it is constituted by 3-(1-methyl-1,2,3,6-tetrahydropyridin-4-yl)-1H-indole, as well as by its salts of addition with pharmaceutically acceptable acids.

10. Pharmaceutical compositions, characterized in that they include, as active principle, one at least of the chemical compounds defined in one of the Claims 1 to 9, as well as an inert pharmaceutical excipient, with the exception of ethanol.